(19) Europäisches Patentamt
European Patent Office
Office européen des brevets

(11) Veröffentlichungsnummer: **0 003 999**
**A2**

(12) **EUROPÄISCHE PATENTANMELDUNG**

(21) Anmeldenummer: 79100525.9

(22) Anmeldetag: 22.02.79

(51) Int. Cl.[2]: **A 01 N 9/20**
A 61 L 13/00, C 11 D 3/48

(30) Priorität: 02.03.78 DE 2808865

(43) Veröffentlichungstag der Anmeldung: 19.09.79 Patentblatt 79/19

(84) Benannte Vertragsstaaten: DE FR GB IT NL

(71) Anmelder: HOECHST AKTIENGESELLSCHAFT
Zentrale Patentabteilung Postfach 80 03 20
D-6230 Frankfurt/Main 80(DE)

(72) Erfinder: Wallhäusser, Karl Heinz, Prof. Dr.
Lessingstrasse 20
D-6238 Hofheim am Taunus(DE)

(72) Erfinder: May, Adolf, Dr.
Dahlienweg 5
D-6238 Hofheim am Taunus(DE)

(72) Erfinder: Bücking, Hans-Walter, Dr.
In den Padenwiesen 30
D-6233 Kelkheim (Taunus)(DE)

(54) **Mikrobiozide Mittel auf der Basis von Alkyl-di-guanidinium-Salzen.**

(57) Mikrobiozide Mittel bestehend aus 10 - 40 Gew.-Teilen des Salzes eines Alkyl-di-guanidins der allgemeinen Formel I

$$H_2N-\underset{\underset{NH}{\|}}{C}-\overset{\overset{R}{|}}{N}-(CH_2)_m-\overset{\overset{H}{|}}{N}-\underset{\underset{NH}{\|}}{C}-NH_2 \qquad (I)$$

wobei R $C_8$-$C_{18}$ Alkyl und m eine Zahl von 2-6 bedeutet. 10 - 40, vorzugsweise 20 - 30 Gewichtsteilen eines Blockpolymerisats der allgemeinen Formel II

$$HO-(CH_2-CH_2-O)_a-(\underset{\underset{CH_3}{|}}{CH}-CH_2-O)_b-(CH_2-CH_2O)_c-H \qquad (II)$$

wobei a, b und c ganze Zahlen darstellen, die so gewählt sind, daß das Molekulargewicht des Blockpolymerisats 1000 bis 16 000 beträgt und der Gewichtsanteil der Polyocyäthylenketten am Gesamtmolekulargewicht 10 - 90 % ausmacht, und der Rest ist Wasser und/oder ein kurzkettiger, wasserlöslicher Alkohol.

EP 0 003 999 A2

Croydon Printing Company Ltd.

HOECHST AKTIENGESELLSCHAFT HOE 78/F 042 Dr.OT/wö

# Mikrobiozide Mittel auf der Basis von Alkyl-di-guanidinium-Salzen

Es ist bereits bekannt, daß Alkyl-diguanidiniumsalze eine gute bakterizide und fungzidie Wirkung besitzen (DE-PS 12 49 457). Wegen der teilweise unbefriedigenden Löslichkeit in Wasser und der schlechten Verträglichkeit besonders mit hartem oder kochsalzhaltigem Wasser ist ihre Einsatzmöglichkeit aber beschränkt.

Um die Löslichkeit und Stabilität in kochsalzhaltigem oder hartem Wasser zu verbessern, ist es bereits bekannt, sie mit quarternären Ammoniumverbindungen bzw. Phosphoniumverbindungen oder mit Fettalkyldiaminsalzen zu kombinieren. Es ist jedoch hierbei von Nachteil, daß die mikrobizide Wirkung der Alkyl-di-guanidinium Salze durch diese Emulgatoren negativ beeinflußt wird.

Es wurde nun gefunden, daß Kombinationen von Alkyl-di-guanidinen in Form von Salzen mit Polyoxäthylen-Polyoxpropylen-Blockpolymerisaten stabile Formulierungen mit verbesserter mikrobizider Wikung ergeben.

Gegenstand der Erfindung sind somit mikrobizide Mittel bestehend aus 10 - 40 vorzugsweise 20 - 30 Gewichtsteilen des Salzes eines alkyl-di-guanidins der allgemeinen Formel I

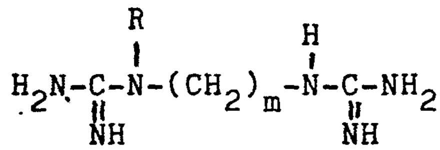 (I)

wobei R $C_8$-$C_{18}$ Alkyl und m eine Zahl von 2-6 bedeutet, 10 - 40, vorzugsweise 20 - 30 Gewichtsteilen eines Blockpolymerisats der allgemeinen Formel II

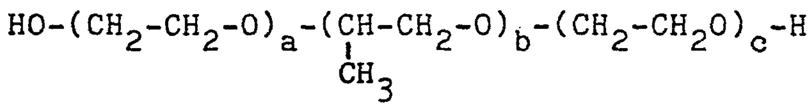 (II)

wobei a, b und c ganze Zahlen darstellen, die so gewählt sind, daß das Molekulargewicht des Blockpolymerisats 1000 bis 16 000 beträgt und der Gewichtsanteil der Polyoxäthylenketten am Gesamtmolekulargewicht 10 - 90 % ausmacht, und der Rest ist Wasser und/oder ein kurzkettiger, wasserlöslicher Alkohol, vorzugsweise Isopropanol.

Die erfindungsgemäß verwendeten Alkyl-di-guanidine lassen sich nach an sich bekannten Verfahren, z.B. durch Reaktion von Diaminen der Formel

$$R - NH - (CH_2)_m - NH_2$$

in der R und m die oben angegebene Bedeutung besitzen, mit Cyanamid oder S-Alkylisothioharnstoff herstellen.

Zur Salzbildung kommen sowohl ein- und mehrwertige anorganische oder organische Säuren in Betracht, beispielsweise Schwefelsäure, Salpetersäure, Phosphorsäure, Ameisensäure oder Salzsäure. Außerdem können zur Salzbildung auch organische Säuren, wie Essigsäure und vor allem höhermolekulare aliphatische Carbonsäure, wie Laurinsäure, Stearinsäure, Ölsäure u.ä., oder deren Gemische eingesetzt werden. An

Stelle einheitlicher Individuen können auch Mischungen der genannten Verbindungen, gegebenenfalls auch mit anderen Microbioziden zusammen verwendet werden.

Diese mikrobioziden Mittel, die durch einfaches Vermischen der angegebenen Komponenten hergestellt werden, zeichnen sich durch eine gute Wasserlöslichkeit aus und sind gut verträglich mit hartem oder kochsalzhaltigem Wasser. Besonders hervorzuheben ist außerdem die verbesserte mikrobiozide Wirkung im Vergleich zu den bisher bekannten Formulierungen mit quaternären Ammoniumverbindungen.

Beispiel

Die folgenden Bestandteile werden zusammengemischt:

25 Teile Laurylpropylendiaminguanidiniummonolaktat
25 Teile Polyoxäthylen-Polyoxypropylen-Blockpolymer Molekulargewicht insgeamt 8000, Molekulargewicht des Polyoxäthylen-Anteils 6300
30 Teile Isopropanol
20 Teile Wasser

Diese Formulierung ist unter den Bedingungen des Schaukeltestes stabil. Sie läßt sich mit Wasser in jedem Verhältnis klar löslich weiterverdünnen.

Die mikrobiozide Wirkung in $\gamma$ /ml wurde nach dem 555-Test der Fytopharmacie in Wageningen/Niederlande durchgeführt. Hierbei wird die Mindestkonzentration in $\gamma$ /ml für die Reduzierung um $10^5$ Keime/ml, ausgehend von $10^7$ Keime/ml festgestellt. Die Kontaktzeit beträgt dabei 5 Minuten.

Als Vergleichsformulierung wurde ein Handelsprodukt in die Untersuchung miteinbezogen, das aus folgenden Komponenten besteht:

25 Teile Laurylpropylendiaminguanidiniumlactat
25 Teile Sojatrimethylammoniumchlorid
50 Teile Lösemittel und Wasser

Bestimmung der bakteriziden und fungiziden Wirkung

Kontaktzeit 5 Minuten

Mindestkonzentration für die Reduzierung um $10^5$ Keime/ml:

| Keimart | Vergleichsprodukt | Beispiel erfindungsgemäß |
|---|---|---|
| Salm. Typh. | 2500 γ/ml | 250 γ/ml |
| Ps. aerug. | ca. 3000 γ/ml | 250 γ/ml |
| Strept. Faec. | 1500 γ/ml | 500 γ/ml |
| Staph. aureus. | 2000 γ/ml | 500 γ/ml |
| Cand. alb. | 3000 γ/ml | 250 γ/ml |

Aus der Tabelle sind die verbesserten mikrobioziden Effekte der erfindungsgemäßen Mischung im Vergleich zu einem Handelsprodukt ersichtlich.
Um eine Keinreduzierung um $10^5$ Keime/ml bei einer Kontaktzeit von 5 Minuten zu erreichen, benötigt man im Vergleich zum handelsüblichen Produkt wesentlich geringere Einsatzkonzentrationen.

Patentanspruch:

Mikrobiozide Mittel bestehend aus 10 - 40 Gew.-Teilen des Salzes eines Alkyl-di-guanidins der allgemeinen Formel I

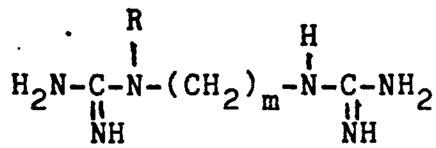

(I)

wobei R $C_8$-$C_{18}$ Alkyl und m eine Zahl von 2-6 bedeutet, 10 - 40, vorzugsweise 20 - 30 Gewichtsteilen eines Blockpolymerisats der allgemeinen Formel II

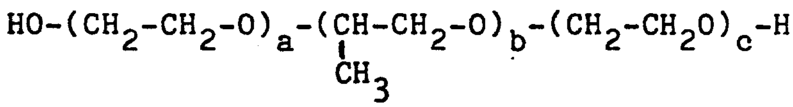

(II)

wobei a, b und c ganze Zahlen darstellen, die so gewählt sind, daß das Molekulargewicht des Blockpolymerisats 1000 bis 16 000 beträgt und der Gewichtsanteil der Polyocyäthylenketten am Gesamtmolekulargewicht 10 - 90 % ausmacht, und der Rest ist Wasser und/oder ein kurzkettiger, wasserlöslicher Alkohol.